# EUROPÄISCHE PATENTSCHRIFT

(11) **EP 0 405 268 B1**
(45) Veröffentlichungstag und Bekanntmachung des Hinweises auf die Patenterteilung: **20.04.1994**
(21) Anmeldenummer: 90111392.8
(22) Anmeldetag: 16.06.1990
(51) Int. Cl.: C07C 253/30, C08C 19/02

(54) **Verfahren zur selektiven Hydrierung nitrilgruppenhaltiger Olefine**
Process for the hydrogenation of olefines containing nitrile groups
Procédé d'hydrogénation d'oléfines contenant des groupes nitriles

(30) Priorität: 29.06.1989 DE 3921263
(43) Veröffentlichungstag der Anmeldung: 02.01.1991
(73) Patentinhaber: BAYER AG, 51368 Leverkusen (DE)
(72) Erfinder: Himmler, Thomas, Dr., D-5000 Köln 80 (DE)

(56) Entgegenhaltungen:
- EP-A- 0 138 141
- EP-A- 0 224 139
- DE-A- 2 034 908
- US-A- 4 170 605
- JOURNAL OF THE CHEMICAL SOCIETY - DALTON TRANSACTIONS, 1985, Seiten 2101-2112, Letchworth, GB; P. MURA: "Complexesof the Platinum Metals. Part 29. 1. Pyridine-2-thiolate Deriatives of Rutheniumand Osmium: ...."
- INORGANICA CHIMICA ACTA, Band 104, 1985, Seiten L5,L6, Elsevier Sequoia S.A., Lausanne, Ch; V. ALTEPARMAKIAN et al.: "Platinum Group Metal Chelates Derived from 2-Mercapto-, 2-Hydroxy- and 2-Amino-pyridines and -pyrimidines"

## Beschreibung

Die Erfindung betrifft ein Verfahren zur Hydrierung von Verbindungen, die mindestens eine olefinische C=C-Doppelbindung und mindestens eine Nitrilgruppe pro Molekül enthalten, unter Erhalt der Nitrilgruppen in Gegenwart von Rutheniumverbindungen.

Verfahren für die selektive Hydrierung nitrilgruppenhaltiger Olefine (d.h. für die Hydrierung der C=C-Doppelbindungen unter Erhalt der Nitrilgruppen) sind bekannt: In der US-PS 3 700 637 werden für die selektive Hydrierung von Dien/(Meth-)Acrylnitril-Copolymerisaten Rhodiumhalogenkomplex-Katalysatoren empfohlen. Die Eignung anderer Metalle wie Platin, Ruthenium, Iridium, Palladium, Rhenium, Kobalt oder Kupfer, homogen oder heterogen, wird angedeutet.

Aus der DE-PS 2 539 132 ist bekannt, daß mit Rhodiumhalogenkomplex-Katalysatoren die vinylischen und die trans-C=C-Doppelbindungen von Butadien/Acrylnitril-Copolymerisaten quantitativ hydriert werden können, wenn Chlorbenzol als Lösungsmittel verwendet wird, während die cis-C=C-Doppelbindungen und die C≡N-Dreifachbindungen erhalten bleiben; in anderen Lösungsmitteln, insbesondere in Ketonen, werden nur niedrige Hydriergrade erzielt.

Die EP-A 134 023 beschreibt die selektive Hydrierung von Nitrilkautschuken in einem aromatischen Lösungsmittel in Gegenwart von Tris-(triphenylphosphan)-rhodium(I)halogenid als Katalysator unter Verwendung von Triphenylphosphan.

Die US-PS 4 464 515 beschreibt die selektive Hydrierung von Copolymerisaten aus konjugierten Dienen und copolymerisierbaren Monomeren in Gegenwart (i) eines einwertigen Rhodiumhydridkomplex-Katalysators, (ii) eines zweiten Liganden und (iii) eines Lösungsmittels für (i), (ii) und das Copolymerisat. Die US-PS 4 503 196 betrifft die gleiche Hydrierung in Abwesenheit des zweiten Liganden (ii).

Da die Rhodiumvorkommen sehr klein und Rhodium nicht nur in der chemischen Industrie, sondern überwiegend in der Elektroindustrie, in der Glas- und Keramikindustrie und in jüngster Zeit ganz besonders in der Automobilindustrie (Abgaskatalysatoren) eingesetzt wird, ist eine Verknappung dieses Edelmetalls in Zukunft nicht auszuschließen. Es war daher wünschenswert, Verfahren zur selektiven Hydrierung nitrilgruppenhaltiger Olefine, insbesondere Verfahren zur selektiven Hydrierung von Nitrilkautschuken, zu entwickeln, die auf Rhodiumkatalysatoren verzichten können. Auch hierzu sind schon verschiedene Vorschläge gemacht worden:

Die EP-A 174 576 beschreibt die selektive Hydrierung nitrilgruppenhaltiger ungesättigter Polymerisate, wobei als Lösungsmittel ein niedermolekulares Keton und als Katalysator eine Verbindung der Formel

RuX [(L¹) (L²)ₙ] (I)

verwendet werden, worin
- X: Wasserstoff, Halogen,
- L¹: Wasserstoff, Halogen, gegebenenfalls substituiertes Cyclopentadienyl
- L²: ein Phosphan, Bisphosphan oder Arsan und
- n: 1, 2 oder 3,
sowie [(L¹) (L²)ₙ] ein Cyclopentadienyl-Bisphosphan bedeuten.

Die US-PS 4 795 788 beschreibt die selektive Hydrierung nitrilgruppenhaltiger ungesättigter Polymerisate in Gegenwart eines Katalysator der Formel I, worin
- X: Wasserstoff, Halogen oder SnCl₃,
- L¹: gegebenenfalls substituiertes Indenyl,
- L²: Phosphan, Bisphosphan oder Arsan und
- n: 1 oder 2
bedeuten.

Die DE-OS 3 540 918 beschreibt die selektive Hydrierung ungesättigter Verbindungen, insbesondere die selektive Hydrierung von Nitrilkautschuk, in Gegenwart eines Katalysators der Formel

RuH₂ₙ L₅₋ₙ (II)

worin
- L: ein Phosphan oder Arsan und
- n: 1 oder 2 bedeuten.

Die DE-OS 3 529 252 empfiehlt die selektive Hydrierung nitrilgruppenhaltiger ungesättigter Polymerisate in Gegenwart eines Katalysator der Formel

RuHₘ (R¹COO)ₙ (Lₚ) (III)

worin
- R¹: Alkyl, Aryl, Cycloalkyl oder Aralkyl,
- L: ein Phosphan oder Arsan,
- m: 0 oder 1,
- n: 1 oder 2 und
- p: 2 oder 3 bedeuten.

Die EP-A 298 386 lehrt die selektive Hydrierung ungesättigter Copolymerisate in Gegenwart verschiedener Rutheniumcarbonyl-Komplexe.

Die als Hydrierkatalysatoren beschriebenen Rutheniumkomplexe sind luftempfindlich und/oder von relativ geringer katalytischer Aktivität, so daß sie in hohen Mengen verwendet werden müssen.

Aufgabe der Erfindung war daher die Bereitstellung eines Rhodium-unabhängigen Verfahrens zur selektiven Hydrierung nitrilgruppenhaltiger Olefine, wobei dieses Verfahren auch auf die Hydrierung von Polymerisaten anwendbar sein sollte; die für dieses Hydrierverfahren notwendigen Komplexe sollten einerseits luftunempfindlich sein und andererseits hohe katalytische Aktivität besitzen.

Überraschenderweise wurde gefunden, daß diese Aufgabe durch eine Hydrierung in homogener Phase in Ketonen als Lösungsmittel erfolgreich gelöst wird, wenn als Hydrierkatalysatoren speziell ausgewählte Rutheniumkomplexe eingesetzt werden.

Gegenstand der Erfindung ist also ein Verfahren zur selektiven Hydrierung nitrilgruppenhaltiger Olefine in einem organischen Lösungsmittel mit Wasserstoff in Gegenwart eines Hydrierkatalysators,
dadurch gekennzeichnet, daß
(i) als organisches Lösungsmittel ein aliphatisches C₃-C₆-Keton und/oder ein cycloaliphatisches C₅-C₆-Keton und
(ii) als Hydrierkatalysator eine Verbindung der Formel

   RuXₘ (L¹)ₙ (CO)ₚ (L²)_{q} (IV)

verwendet werden, worin
- X: Wasserstoff, Halogen (Fluor, Chlor, Brom) oder SnCl₃,
- L¹: einen anionischen Rest der Formel 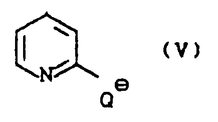
worin
- Q: Sauerstoff, Schwefel oder NH bedeutet,
- L²: Phosphan, Bisphosphan oder Arsan,
- m: Null, 1 oder 2,
- n: 1 oder 2,
- p: Null oder 1 und
- q: 1 oder 2 bedeuten.

L²-Liganden sind z.B. solche, die den Formeln 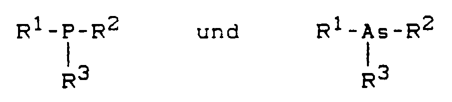
entsprechen, in denen R¹, R² und R³ gleich oder verschieden sein können und (gegebenenfalls substituierte) Alkyl-, Cycloalkyl-, Aryl- oder Aralkylreste bedeuten.

Alkylreste R¹ bis R³ sind beispielsweise geradkettige oder verzweigte, gesättigte Kohlenwasserstoffreste mit 1 bis 20, bevorzugt 1 bis 12, besonders bevorzugt 1 bis 6 C-Atomen.

Cycloalkylreste R¹ bis R³ sind beispielsweise cyclische, gesättigte Kohlenwasserstoffreste mit 5 bis 12, bevorzugt 5 bis 7 C-Atomen.

Arylreste R¹ bis R³ sind beispielsweise aromatische Kohlenwasserstoffreste aus der Benzolreihe mit 6 bis 18, bevorzugt 6 bis 12 C-Atomen.

Aralkylreste R¹ bis R³ sind beispielsweise durch Aryl substituierte Alkylreste, die im aliphatischen Teil aus einem geradkettigen oder verzweigten Kohlenwasserstoffrest mit 1 bis 6 C-Atomen und im aromatischen Teil aus einem Rest der Benzolreihe, vorzugsweise Phenyl, bestehen.

Die vorstehend erläuterten Alkyl-, Cycloalkyl-, Aryl- und Aralkylreste können gegebenenfalls durch C₁-C₆-Alkyl, Hydroxy, C₁-C₆-Alkoxy, C₁-C₆-Carbalkoxy, Fluor oder Chlor substituiert sein.

Bevorzugte L²-Liganden sind Triphenylphosphan, Diethylphenylphosphan, Tritolylphosphan, Trinaphthylphosphan, Diphenylphosphan, Tributylphosphan, Tris-(trimethoxyphenyl)-phosphane, Bis-(trimethylphenyl)-phenylphosphane, Bis-(trimethoxyphenyl)-phenylphosphane, Trimethylphenyl-diphenylphosphane, Trimethoxyphenyldiphenyl-phosphane, Tris-(dimethylphenyl)-phenylphosphane, Bis-(dimethoxyphenyl)-phenylphosphane, Dimethylphenyldiphenylphosphane, Dimethoxyphenyldiphenylphosphane, Triphenylarsan, Ditolylphenylarsan, Tris-(4-ethoxy)-arsan, Diphenylcyclohexylarsan, Dibutylphenylarsan und Diethylphenylarsan.

Weitere Beispiele für L²-Liganden sind Bisphosphane der Formel 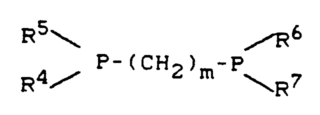
in der m für eine ganze Zahl von 1 bis 10 steht und die Reste R⁴ bis R⁷ die Bedeutung von R¹ haben.

Beispiele für Bisphosphane sind 1,2-Bis-(diphenylphosphano)-ethan, 1,2-Bis-(dianisylphosphano)-ethan, bevorzugt 1,3-Bis-(diphenylphosphano)-propan und insbesondere 1,4-Bis-(diphenylphosphano)-butan,

Bevorzugter Ligand L¹ ist der Rest V mit Q = Sauerstoff.

Besonders bevorzugter Katalysator ist Bis-(2-hydroxypyridinato)-bis-(triphenylphosphan)-ruthenium (II).

Die erfindungsgemäß zu verwendenden Hydrierkatalysatoren und Verfahren zu ihrer Herstellung sind entweder bekannt oder die Herstellung kann in anloger weise zu bekannten Herstellungverfahren erfolgen; V. Alteparmakian, P. Mura, B.G. Olby und S.D. Robinson, Inorg. Chim Acta 104 (1985) L5.

Für das erfindungsgemäße Verfahren geeignete nitrilgruppenhaltige Olefine, umfassen z.B. Acrylnitril, Methacrylnitril und Cyclohex-3-en-nitril.

Für das erfindunggsgemäße Verfahren bevorzugte nitrilgruppenhaltige Olefine sind Polymere mit als Zahlenmittel bestimmten mittleren Molekulargewichte M̅_{w} von 500 bis 500.000, vorzugsweise 1.000 bis 200.000 und insbesondere 30.000 bis 150.000. Die Molekulargewichte M̅_{w} können durch Gelpermeationschromatographie mit Polystyrol als Standard bestimmt werden.

Bevorzugte nitrilgruppenhaltige olefinisch ungesättigte Polymere umfassen Copolymerisate aus 85 bis 50 Gew.-%, bevorzugt 82 bis 55 Gew.-% mindestens aus einem konjugierten Dien, 15 bis 50 Gew.-%, vorzugsweise 18 bis 45 Gew.-% mindestens einem ungesättigten Nitril und 0 bis 10 Gew.-%, bevorzugt 0 bis 8 Gew.-% mindestens einem weiteren, mit konjugierten Dienen und ungesättigten Nitrilen copolymerisierbaren Monomeren.

Als konjugierte Diene kommen z.B. Butadien-1,3,2-Methylbutadien-1,3, 2,3-Dimethylbutadien-1,3 und Pentadien-1,3, als ungesättigte Nitrile Acrylnitril und Methacrylnitril in Frage.

Als weitere Monomere kommen Vinylaromaten wie Styrol, o-, m- oder p-Methylstyrol, Ethylstyrol, Vinylnaphthalin und Vinylpyridin, α,β-ungesättigte Monocarbonsäuren mit 3 bis 5 C-Atomen wie Acrylsäure, Methacrylsäure und Crotonsäure sowie α,β-ungesättigte Dicarbonsäuren mit 4 bis 5 C-Atomen wie Malein-, Fumar-, Citracon- und Itaconsäure, ferner Vinylchlorid, Vinylidenchlorid, N-Methylolacrylamid und Vinylalkylether mit 1 bis 4 C-Atomen im Alkylteil in Betracht.

Die bevorzugtesten nitrilgruppenhaltigen olefinisch ungesättigten Polymeren sind Nitrilkautschuke mit Glasübergangstemperaturen unter 0°C, vorzugsweise unter -10° C. Bevorzugte Nitrilkautschuke sind Butadien/Acrylnitril-Copolymerisate mit einem Gehalt an copolymerisiertem Acrylnitril von 5 bis 60, vorzugsweise 10 bis 50 Gew.-%. Sie besitzen in der Regel Mooney-Viskositäten (DIN 53 523), von 1o bis 150, vorzugsweise von 25 bis 80 (ML 1+4/100° C).

Sowohl die als Ausgangsprodukte verwendeten nitrilgruppenhaltigen Olefine als auch die daraus hergestellten Hydrierungsprodukte sollten in dem verwendeten organischen Lösungsmittel löslicher sein. Bevorzugte organische Lösungsmittel sind deshalb Aceton, Butanon, Pentanone, Cyclopentanon und Cyclohexanon.

Die Konzentration an Katalysator, (berechnet als Ruthenium und bezogen auf zu hydrierendes nitrilgruppenhaltiges Olefin) beträgt im allgemeinen 10 bis 1000, vorzugsweise 40 bis 600 ppm. Die Konzentration an nitrilgruppenhaltigem Olefin, bezogen auf die Gesamtlösung, beträgt im allgemeinen 1 bis 90, vorzugsweise 5 bis 40 Gew.-%.

Die Hydrierung kann zweckmäßigerweise bei 80 bis 200° C, vorzugsweise bei 100 bis 180° C, insbesondere bei 120 bis 160° C, und bei 20 bis 350 bar, vorzugsweise bei 30 bis 250 bar Wasserstoffdruck durchgeführt werden.

Die Hydriergrade (Prozentsatz der hydrierten C=C-Doppelbindungen, bezogen auf die Gesamtzahl der ursprünglich im Ausgangsprodukt vorhandenen C=C-Doppelbindungen) können bis 100 % betragen. Die Hydrierung kann jedoch erforderlichenfalls vorher abgebrochen werden. Bevorzugt werden Produkte mit Hydriergraden von über 80 % nach dem erfindunggsgemäßen Verfahren hergestellt. Der Hydriergrad kann mittels IR-Spektroskopie ermittelt werden.

Nach der Hydierung können die Reaktionsprodukte mit Hilfe üblicher Methoden aus der Lösung abgetrennt werden. Übliche Methoden umfassen z.B. Destillation, Eindampfen (gegebenenfalls unter verminderten Druck), Einblasen von Wasserdampf und Zugabe eines Fällungsmittels (Nichtlösungsmittels). Zur Entfernung von Restlösungsmittel oder Wasser kann sich eine Trocknung anschließen.

Sofern es sich bei den Hydrierungsprodukten um Kautschuke handelt, können sie in üblicher Weise durch Peroxid- oder Schwefelvulkanisation oder durch Strahlenvernetzung vulkanisiert werden. Aufgrund ihrer ausgezeichneten Wetter-, Ozon-, Öl- und Heißluftbeständigkeit gegenüber kaltem Klima können diese hydrierten Polymere bekanntlich für hochwertige Gummiartikel wie Dichtungen, Schläuche, Membranen, für Kabelisolationen und -mäntel eingesetzt werden.

### Beispiele 1 und 2

Das erfindungsgemäße Verfahren wird am Beispiel der Hydrierung von Cyclohex-3-en-nitril beschrieben:

### Beispiel 1

In einem Rühr-Autoklaven aus rostfreien Stahl von 0,7 l Inhalt wird unter Stickstoff eine Lösung von 0,51 Mol Cyclohex-3-en-nitril und 0,163 mMol Ru(pyO)₂ (PPh₃)₂ (pyO = C₅H₄N-2-O^{⊖}) (≙ 0,03 Mol-% bzw. 300 ppm Ru) in Aceton vorgelegt. Man drückt 30 bar Wasserstoff auf, heizt auf 130° C, erhöht den Wasserstoffdruck auf 90 bis 100 bar und hält diesen Druck 4 Stunden. Nach Abkühlen wird entspannt und die Reaktionslösung gaschromatographisch untersucht. Der Umsatz an Cyclohex-3-en-nitril ist über 99 %.

### Beispiel 2

Es wird analog Beispiel 1 mit dem Unterschied vorgegangen, daß Ru(pyNH)₂ (PPh₃)₂ (pyNH = C₅H₄N-2-NH^{⊖}) als Katalysator eingesetzt wird. Der Umsatz an Cyclohex-3-en-nitril ist über 99 %.

### Beispiel 3 bis 6

Diese Beispiele beschreiben die Hydrierung eines Butadien/Acrylnitril-Copolymerisats:

### Beispiel 3

In 1,6 kg Stickstoff-gespülten Aceton werden 0,257 g Ru(pyO)₂ (PPh₃)₂ (= 200 ppm Ru) gelöst. Anschließend löst man 160 g eines statistischen Butadien/Acrylnitril-Copolymerisats mit 34,9 Gew.-% copolymerisiertem Acrylnitril und einer Mooney-Viskosität von 29 (ML 1+4 100° C). Diese Lösung wird in einem Rühr-Autoklav aus rostfreien Stahl von 3 l Inhalt transferiert; man drückt 80 bar Wasserstoff auf, erhitzt auf 135° C, erhöht den Druck auf 170 bis 180 bar und hält diesen 6 Stunden. Der IR-spektroskopisch bestimmte Hydriergrad ist über 98 %.

### Beispiel 4

160 g des statistischen Butadien/Acrylnitril-Copolymeren aus Beispiel 3 werden in 1,6 kg Stickstoff-gespülten Aceton gelöst. Diese Lösung wird in einen 3 l Autoklaven überführt. Man drückt 40 bar Wasserstoff auf, erwärmt auf 100° C und erhöht dann den Wasserstofdruck auf 100 bar. Nun wird eine Lösung von 0,386 g Ru(pyO)₂ (PPh₃)₂ (≙ 300 ppm Ru) in 30 ml Chlorbenzol mittels Wasserstoffüberdruck in den Autoklaven gepreßt. Die Reaktionstemperatur wird auf 135°C und der Druck auf 180 bar erhöht. Nach 4 Stunden ist der Hydriergrad über 98 %.

### Beispiel 5

Man verfährt wie in Beispiel 4, setzt jedoch nur 0,257 g Ru(pyO)₂ (PPh₃)₂ (≙ 200 ppm Ru) ein. Der Hydriergrad nach 6 Stunden beträgt 80 %.

### Beispiel 6

Man verfährt entsprechend Beispiel 4, setzt jedoch nur 0,193 g Ru(pyO)₂ (PPh₃)₂ (≙ 150 ppm Ru) ein. Der Hydriergrad nach 6 Stunden beträgt 70 %.

## Patentansprüche

1. Verfahren zur selektiven Hydrierung nitrilgruppenhaltiger Olefine in einem organischen Lösungsmittel mit Wasserstoff in Gegenwart eines Hydrierkatalysators,
dadurch gekennzeichnet, daß
(i) als organisches Lösungsmittel ein aliphatisches C₃-C₆-Keton und/oder ein cycloaliphatisches C₅-C₆-Keton und
(ii) als Hydrierkatalysator eine Verbindung der Formel
RuXₘ (L¹)ₙ (CO)ₚ (L²)_{q} (IV)
verwendet werden, worin
X Wasserstoff, Halogen (Fluor, Chlor, Brom) oder SnCl₃,
L¹ einen anionischen Rest der Formel 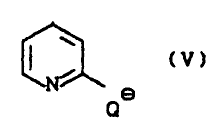
worin
Q Sauerstoff, Schwefel oder NH bedeutet,
L² Phosphan, Bisphosphan oder Arsan,
m Null, 1 oder 2,
n 1 oder 2,
p Null oder 1 und
q 1 oder 2 bedeuten.

2. Verfahren nach Anspruch 1, wobei L² ein Triphenylphosphan bedeutet.

3. Verfahren nach Ansprüchen 1 und 2, wobei p Null und q 2 bedeuten.

4. Verfahren nach Ansprüchen 1 und 2, wobei m Null, n 2, p Null und q 2 bedeuten.

5. Verfahren nach Ansprüchen 1 bis 4, wobei als Lösungsmittel Aceton und/oder Butanon verwendet werden.

6. Verfahren nach Ansprüchen 1 bis 5, wobei man bei einem Wasserstoffdruck von 20 bis 350 bar arbeitet.

7. Verfahren nach Ansprüchen 1 bis 6, wobei man bei Temperaturen von 80 bis 200° C arbeitet.

## Claims

1. A process for the selective hydrogenation of olefins containing nitrile groups with hydrogen in an organic solvent in the presence of a hydrogenation catalyst,
characterized in that
(i) the organic solvent used is an aliphatic C₃₋₆ ketone and/or a cycloaliphatic C₅₋₆ ketone and
(ii) the hydrogenation catalyst used is a compound corresponding to the formula:
RuXₘ (L¹)ₙ (CO)ₚ (L²)_{q} (IV)
in which
X is hydrogen, halogen (fluorine, chlorine, bromine) or SnCl₃,
L¹ is an anionic group corresponding to the formula: 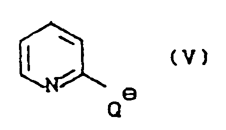 where Q is oxygen, sulfur or NH,
L² is phosphane, bisphosphane or arsane,
m = 0, 1 or 2,
n = 1 or 2,
p = 0 or 1 and
q = 1 or 2.

2. A process as claimed in claim 1, L² being a triphenyl phosphane.

3. A process as claimed in claims 1 and 2, p being 0 and q being 2.

4. A process as claimed in claims 1 and 2, m being 0, n being 2, p being 0 and q being 2.

5. A process as claimed in claims 1 to 4, characterized in that acetone and/or butanone is used as the solvent.

6. A process as claimed in claims 1 to 5, characterized in that it is carried out under a hydrogen pressure of 20 to 350 bar.

7. A process as claimed in claims 1 to 6, characterized in that it is carried out at temperatures of 80 to 200°C.

## Revendications

1. Procédé d'hydrogénation sélective d'oléfines portant des groupes nitrile dans un solvant organique, avec de l'hydrogène en présence d'un catalyseur d'hydrogénation,
caractérisé en ce qu'on utilise
(i) comme solvant organique, une cétone aliphatique en C₃ à C₆ et/ou une cétone cycloaliphatique en C₅ ou C₆ et
(ii) comme catalyseur d'hydrogénation, un composé de formule
RuXₘ (L¹)ₙ (CO)ₚ (L²)_{q} (IV)
dans laquelle
X représente l'hydrogène, un halogène (fluor, chlore, brome) ou un groupe SnCl₃,
L¹ est un reste anionique de formule 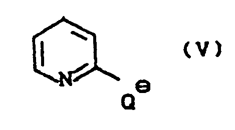
dans laquelle
Q représente de l'oxygène, le soufre ou un groupe NH,
L² est un phosphane, un bisphosphane ou un arsane,
m a la valeur zéro, 1 ou 2,
n a la valeur 1 ou 2,
p a la valeur zéro ou 1 et
q a la valeur 1 ou 2.

2. Procédé suivant la revendication 1, dans lequel L² est un triphénylphosphane.

3. Procédé suivant les revendications 1 et 2, dans lequel p est égal à zéro et q est égal à 2.

4. Procédé suivant les revendications 1 et 2, dans lequel m est égal à zéro, n est égal à 2, p est égal à 0 et q est égal à 2.

5. Procédé suivant les revendications 1 à 4, dans lequel on utilise comme solvants l'acétone et/ou la butanone.

6. Procédé suivant les revendications 1 à 5, dans lequel on opère à une pression d'hydrogène de 20 à 350 bars.

7. Procédé suivant les revendications 1 à 6, dans lequel on opère à des températures de 80 à 200°C.
